# EUROPEAN PATENT SPECIFICATION

(11) **EP 0 504 289 B1**
(45) Date of publication and mention of the grant of the patent: **15.09.1999**
(21) Application number: 91901398.7
(22) Date of filing: 07.12.1990
(51) Int. Cl.: C12Q 1/02, C12N 5/00

(54) **CELL-CYCLE-DEPENDENT REGULATION OF PHOSPHORYLATION OF HUMAN RETINOBLASTOMA GENE PRODUCT**
ZELLZYKLUS-ABHÄNGIGE REGULATION DER PHOSPHORYLIERUNG DES MENSCHLICHEN RETINOBLASTOMA-GENPRODUKTES
REGULATION DEPENDANT DU CYCLE DE CELLULE DE PHOSPHORYLATION D'UN PRODUIT GENETIQUE DE RETINOBLASTOME HUMAIN

(30) Priority: 07.12.1989 US 447282
(43) Date of publication of application: 23.09.1992
(73) Proprietor: CANJI, Inc., San Diego, CA 92121 (US)
(72) Inventor: FUNG, Yuen, Kai, Los Angeles, CA 90039 (US)
(74) Representative: Wilkinson, Stephen John
(86) International application number: US9007216
(87) International publication number: WO9109114

(56) References cited:
- US-A- 4 694 023
- CELL vol. 62, no. 1, 13 July 1990, CAMBRIDGE, NA US pages 175 - 185 MARIKKI LAIHO ET AL. 'GROWTH INHIBITION BY TGF-BETA LINKED TO SUPPRESSION OF RETINOBLASTOMA PROTEIN PHOSPHORYLATION' THE WHOLE ARTICLE
- Molecular and Cellular Biology Volume 8, Number 8, issued August 1988, COFFEY et al. "Selective Inhibition of Growth-Related Gene Expression in Murine Keratinocytes by Transforming Growth Factor B" pages 3088-3093. See page 3088, first paragraph and page 3091 "Discussion", first paragraph.
- Experimental Cell Research, Volume 162 Number 2, issued February 1986, KAMECH, et. al. "Butyrate Converts Rat 3T3 Fibroblasts into Giant Cells" pages 326-334. See page 331, first and second paragraphs, Tables 1 and 2.

## Description

### Field of the Invention

The present invention relates to a method of identifying drugs which inhibit abnormal cell proliferation, to a method of determining whether an abnormally proliferating cell will be growth inhibited in response to a drug and to a method of determining the effectiveness of a drug for the treatment of a cell proliferative disease.

### Background of the Invention

Deprived of growth factors, actively proliferating cells become arrested at the Gₒ phase of the cell cycle. Conversely, quiescent cells are induced to proliferate when provided with the proper growth factors. Exposure of quiescent cells to growth stimuli leads to quantitative and qualitative changes in the expression of many specific genes and their products. For example, exposure of resting cells to platelet derived growth factor (PDGF) (Wahl, et al., Mol. Cell. Biol. 9: 2934 (1989); Meisenhelder, et. al. Cell, 57: 1109 (1989)) or epidermal growth factor (Wahl et. al., Proc. Natl. Acad. Sci. USA 86: 1568 (1989); Margolis, et al., Cell 57: 1101 (1989)) leads to the activation of the tyrosine kinase activity of their receptors which may then directly phosphorylate phospholipase C. This, in turn, may result in other biochemical responses such as the elevation of intracellular calcium ions, the hydrolysis of phosphoinositides, the modulation of protein phosphorylation, and the induction of synthesis of new mRNA [see Deuel, Annu. Rev. Cell Biol. 3: 443 (1987)]. Because some of these transcriptionally activated genes are protooncogenes, studies on the control of cell proliferation have mainly focused on the isolation and characterization of similar genes whose expression is activated in the early hours of the proliferative response (Cochran, et. al., Science 226: 1080 (1984); Thompson, et. al., Nature 319: 374 (1986); Lau and Nathans, Proc. Natl. Acad. Sci. (USA) 84: 1182 (1987); Ryter, Proc. Natl. Acad. Sci. (USA) 85: 1487 (1988)).
A number of genes which express specifically in the quiescent state have also been isolated (Schneider, et. al., Cell 14, 787 (1988); Bedard, et. al., Simmons and Erikson, Mol. Cell. Biol. 9, 1371 (1989)). The expression of these genes is repressed when a cell is exposed to growth stimuli. Therefore, down regulation of specific genes may also be important for the mitogenic response, by the removal of blocks to cell proliferation.

The human retinoblastoma gene, RB1, is the prototype of a class of genes in which the inactivation of both alleles is thought to be essential for neoplastic growth. The isolation of RB1 has allowed a confirmation at the molecular level of the loss of the gene in retinoblastoma and clinically related tumors such as osteosarcomas (Friend et. al., Nature 323,643 (1986); Fung et al., Science 236: 1657 (1987); Lee et. al., Science 235: 1394 (1987)). Moreover, analysis using the RB1 cDNA probe and antibodies to its gene product (Rb) has revealed the loss of RB1 in a large number of adult tumors such as small cell lung carcinoma (Harbour et. al., Science 241, 353 (1988); Yokota et al., Oncogene 3, 471 (1988)) and breast tumors (T'Ang, et. al., Science 242, 263 (1988)). RBl's important function in the control of neoplastic growth is supported by the fact that Rb forms a physical association with the transformation proteins of adenovirus E1A (Whyte et. al., Nature 334, 124(1988)), simian virus 40 (SV40) large T antigen (Decaprio et. al., Cell 54, 275 (1988)) and the E7 protein of human Papillomavirus Type 16 (Dyson, et. al., Science 243, 934 (1989)).

Cell 58, September 1989, pages 1097-1105 describes the cell cycle-dependent phosphorylation of Rb protein. Cell, 58, September 1989, pages 1085-1095 also discloses the phosphorylation of Rb protein during specific phases of the cell cycle. Cell, 62, July 1990, pages 175-185 teaches that growth inhibition by TGF-β is linked to suppression of Rb protein phosphorylation.

### Brief Description of the Drawings

Figure 1A shows the immunoprecipitation of RB with RB 1-AB 20 by SDS-PAGE.

Figure 1B demonstrates the analysis of the steady-state of RB by flow cytometry.

Figure 1C shows an SDS-PAGE analysis of Rb degradation in pulse-chase experiments.

Figure 2 demonstrates the phosphorylation of Rb in cell under different growth conditions.

Figure 3 demonstrates the Rb phosphorylation in HL-60A cells induced to differentiate by various chemicals.

Figure 4 shows (by underlining) the 11 potential phosphorylation sites in the Rb protein.

Figure 5 demonstrates the ability of antibodies to distinguish between phosphorylated and underphosphorylated forms of the Rb protein. In lane 1, the Rb protein is immunoprecipitated with antibody OS3. In lane 2, the Rb protein is immunoprecipitated with antibody OS1.

### Summary of the Invention

The inactivation of Rb1 is associated with tumorigenesis.

According to the present invention, there is provided a method of identifying drugs which inhibit abnormal cell proliferation comprising incubating said drug with abnormally proliferating cells containing Rb protein for a period of time sufficient to allow phosphorylation of said Rb protein, and measuring the extent of underphosphorylation of said Rb protein in said cells. Preferably, the incubation period is 12 hours.

The present invention also provides a method of determining whether an abnormally proliferating cell will be growth inhibited in response to a drug comprising incubating said drug with abnormally proliferating cells containing Rb protein for a period of time sufficient to allow phosphorylation of said Rb protein, and measuring the extent of underphosphorylation of said Rb protein in said cells.

In another embodiment, the present invention relates to a method for determining the effectiveness of a drug for the treatment of a cell proliferative disease comprising:
a) treating a portion of a cell sample, obtained from an individual prior to treatment with the drug, with said drug in vitro;
b) measuring the level of Rb protein phosphorylation in the portion of the cell sample treated in step (a);
c) measuring the level of Rb protein phosphorylation in an untreated portion of the cell sample;
d) comparing the level of Rb protein phosphorylation from said cell sample portion of step (b) with the level of Rb protein phosphorylation from said cell sample portion of step (c),
wherein a drug which inhibits Rb protein phosphorylation is thereby assessed to be effective in treating cell proliferative disease.

Growth factor-induced activation of the expression of genes that have a positive effect on cell growth appears to be important in the control of cell proliferation. However, down modulation of the expression of genes that are inhibitory towards cell growth may also be important in growth regulation. The fact that inactivation of RB1 is correlated with tumor development suggests that this gene participates in the control of cell proliferation. We have demonstrated that newly synthesized Rb in cells at the G₀ and G₁ phases is underphosphorylated and that newly synthesized Rb is phosphorylated at multiple sites only in cells at the G₁/S boundary and in the S phase. Our observations are consistent with the interpretation that the underphosphorylated form of Rb is the active form and that this form of Rb is rendered inactive by phosphorylation at one or more critical sites before the initiation of DNA synthesis. The SV40 large T antigen can only bind to, and presumably inactivate, the underphosphorylated form of the Rb (Ludlow et. al., Cell 56, 57 (1989)). Thus, the inactivation of Rb by phosphorylation may be an obligatory event for cells to traverse the G₁/S boundary. Control of cell proliferation may therefore be exerted at the level of Rb inactivation. It is unclear whether the observed variation in the abundance of the phosphorylated Rb species is due to a change in kinase activity, phosphatase activity, or both.

### Detailed Description of the Invention

### Example 1

The regulation of the expression of Rb in cells under various growth conditions was examined.

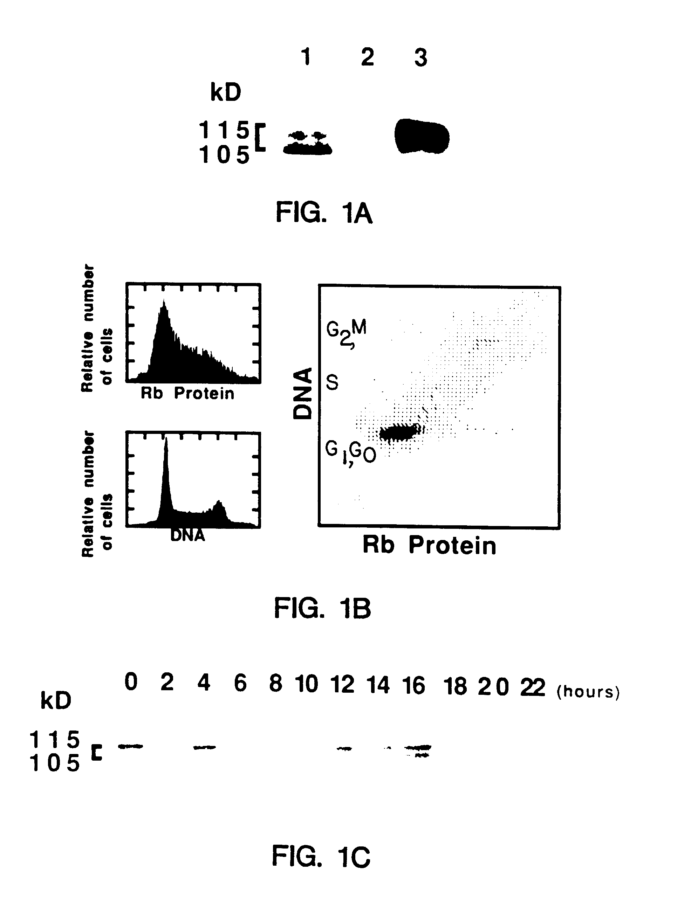

Peptides representing the most hydrophilic regions of Rb were synthesized and used to immunize rabbits and Balb/c mice for the production of polyclonal and monoclonal antibodies. For preparation of antibodies against Rb, peptides synthesized according to the protein sequence deduced from the RB1 cDNA sequence were used to immunize young New Zealand rabbits. For Rb-1-AB 16 the peptide (P4) used.was CEEIYLXNKDLDARLELDHDR (amino acids 322 to 341). For Rb-1-AB 20 the peptide (P5) used was CEGSNPPKPLKKLRFDIEGSDEAD (amino acids 864 to 886). For RB-1-AB Al, the peptide (P2) used was CRMQKQKMNDSMDTSNKEEK (amino acids 910 to 928). Fig. 1 (A) demonstrates the immunoprecipitation of Rb with Rb 1-AB 20. An actively proliferating random population of VM-CUB-3 (4 x 10⁶ cells) was incubated in Dulbecco's Modified Eagles Medium (DMEM, containing 5% dialyzed fetal calf serum (FCS) without methionine (lane 1 and 2) or phosphate (lane 3) for 1 hour and then labeled with 1 ml of DMEM + 5% dialyzed fetal calf serum (FCS) containing 300 uCi of [³⁵S]-methionine (1000 Ci/mmol) (lane 1 and 2 ) or 300 uCi of [³²P] orthophosphate (285 Ci/mg) (lane 3) for 2 hours at 37° C. Cells were then washed twice with phosphate-buffered saline (PBS) and proteins were extracted with 0.5 ml of EBC (40 mM tris-HCl [pH 8.0]), 120 mM NaCl, 0.5% NP-40, 2 ug/ml each of aprotinine, pepstatin and leupeptin, and 100 ug/ml of phenylmethylsulfonyl fluoride at 0° C for 1 hour. The cell lysates were clarified by centrifugation at 15,000 rpm for 10 min. at 4° C. The supernatant was incubated with 5-10 ul of Rb 1-AB 20 antiserum (lanes 1 and 3) or Rb 1-AB 20 preincubated with 100 ug of the peptide P5 at 4° C for 2 hours (lane 2). The immunocomplex was collected on protein A-agarose (Calbiochem) and washed 5 times with EBC. The immunoprecipitated proteins were eluted by heating for 1 minute at 90° C in sample buffer (62.5 mM tris-HCl (pH 6.8), 5 % B-mercaptoethanol, 2.3% SDS, 10% glycerol, and 0.001 % bromophenol blue) and separated by SDS-PAGE. The gel was fixed and exposed to X-ray film.

Figure 1B shows the analysis of the steady-state level of Rb by flow cytometry. Exponentially proliferating HL-60 cells (2 x 10⁶) were suspended in 0.1 ml PBS at 4° C. and fixed by adding 0.9 ml of methanol cooled to -80° C. The fixed cells were resuspended at 4° C. in 120 ul of PBS-NGS (50% PBS, 50% normal goat serum, 0.002 % Triton X-100 and 10 ul of Rb 1-AB 20). The suspension was incubated for 1.5 hours at 37° C with periodic mixing. The suspension was cooled on ice for 2 min. and then 150 ul of PBS was added. The cells were incubated for 30 min. at 4° C with periodic mixing. For secondary staining, the cells were resuspended in 195 ul of PBS-NGS and 5 ul of FITC-GAR (fluorescein isothiocyanate-conjugated goat antibody to rabbit IgG). The suspension was incubated for 1 hour at 37° C, cooled for 2 min. on ice, and 150 ul of PBS-NGS was then added. This washing step was repeated once and the cells were then treated with Rnase A (5 ug/ml) for 30 min. at 37° C. The cells were finally stained with propidium iodide (5 ug/ul) and analyzed by flow cytometry. Flow cytometry was performed using an argon ion laser flow cytometer (Profile Model, Coulter Electronics). Excitation was at 488 nm. Green emission was collected with a Photomultiplier masked with a 525 nm band pass filter for measuring Rb content and red fluorescence was collected with a Photomultiplier masked with a 610 nm long pass filter. Events (2 X 10⁴) were collected at a sample flow rate of 10 ul/minute. The univariate histograms show the distribution of the relative number of cells (vertical axis) as a function of cellular Rb or DNA content (horizontal axis). The Rb content versus the DNA content is shown as a dot density plot. Cell cycle phases are shown in the plot. Figure 1C demonstrates the measurement of Rb degradation by pulse-chase experiments. SW613 cells were pulse labeled as described above with 300 uCi of [³⁵S]-methionine (1000 Ci/mmol) for 90 min. and chased in RPMI 1640 plus 10% FCS for 22 hours. Replicate samples of Rb protein were extracted, immunoprecipitated and separated by SDS-PAGE as described in above at two hour intervals during the chase period.

### Example 2

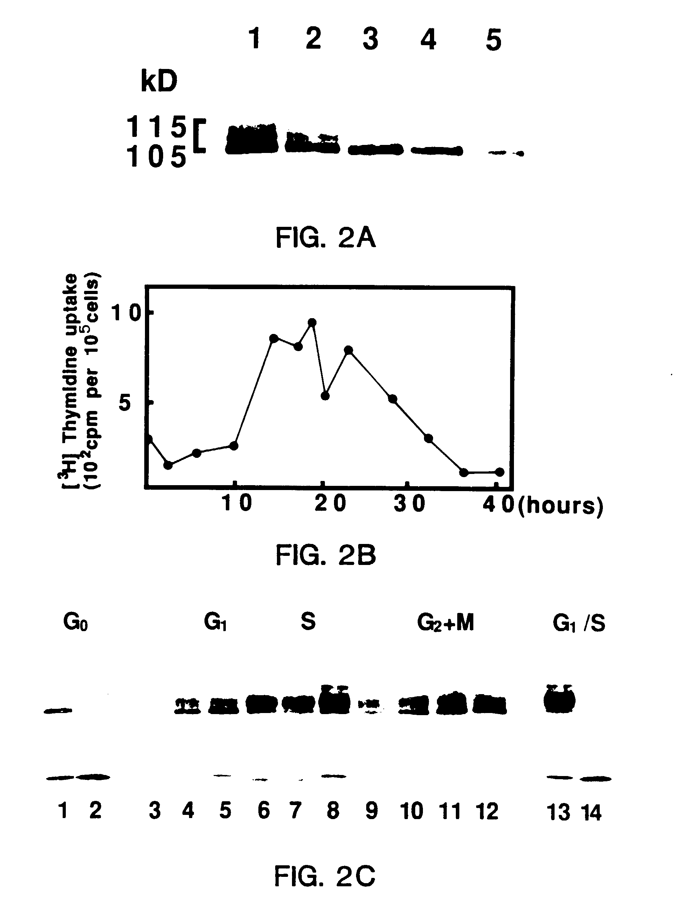

The regulation of phosphorylation of Rb in cells under different growth conditions is demonstrated in Figure 2. An actively growing, random population of VM-CUB-3 was grown in the absence of serum in RPMI 1640 medium for 4 days. During these 4 days, cells were labeled with 300 uCi of [³⁵S]-methionine (1000 Ci/mmol). Rb protein was extracted and immunoprecipitated after 0 (Figure 2A, lane 1), 24 (Figure 2A, lane 2), 48 (Figure 2A, lane 3), 72 (Figure 2A, lane 4) and 96 hours (Figure 2A, lane 7) and the samples analyzed as was described above.

### Example 3

SW613 cells made quiescent by serum-starvation for 2 days were stimulated to grow by replenishing the medium with 10% fetal calf serum. At regular intervals after serum was added to the cells, DNA synthesis was measured by [³Hl-thymidine uptake as described by Macher et. al., Exp. Cell Res. 117: 95 (1978). Duplicate cultures of cells at each time point were labeled with [³⁵S]-methionine as described in Example 1A. To more precisely analyze the timing of onset of multiple phosphorylation, cells synchronized by serum-starvation were arrested at the G₁/S boundary by treatment with aphidicolin (5ng/ml) for 24 hours (Figure 2C, lane 13). After 24 hours of treatment, these cells were labeled for 2 hours with 300 Uci of [³⁵S]-methionine (1000 Ci/mmol) in the presence of aphidicolin and analyzed as described in Example 1A. The results are shown on Figure 2C (Lane 1, Go phase; lane 2, same as lane 1 except that the antiserum Rbl-AB20 was preincubated at 4 C for 2 hours with 100ug of peptide P5; lanes 3 and 4, early G₁ phase; lane 5, late G, early S phase; lanes 6 to 10, S phases; lanes 11 and 12, G₂+M phases; lane 13, G₁/S boundary; lane 14, same as lane 13 except that Rb 1-AB-20 was preincubated at 4° C for 2 hours with 100ug of peptide P5.

### Example 4

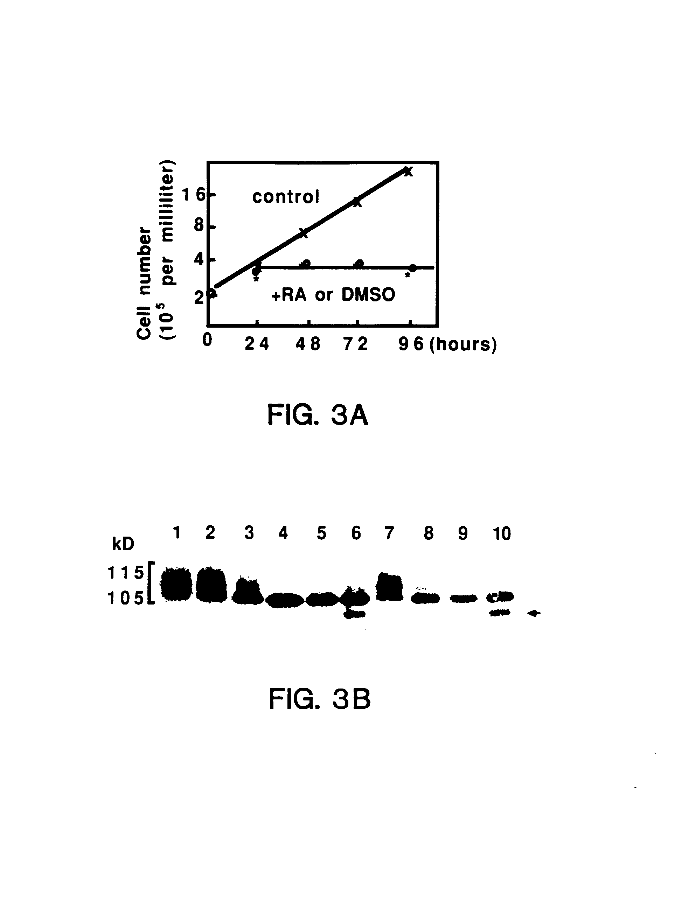

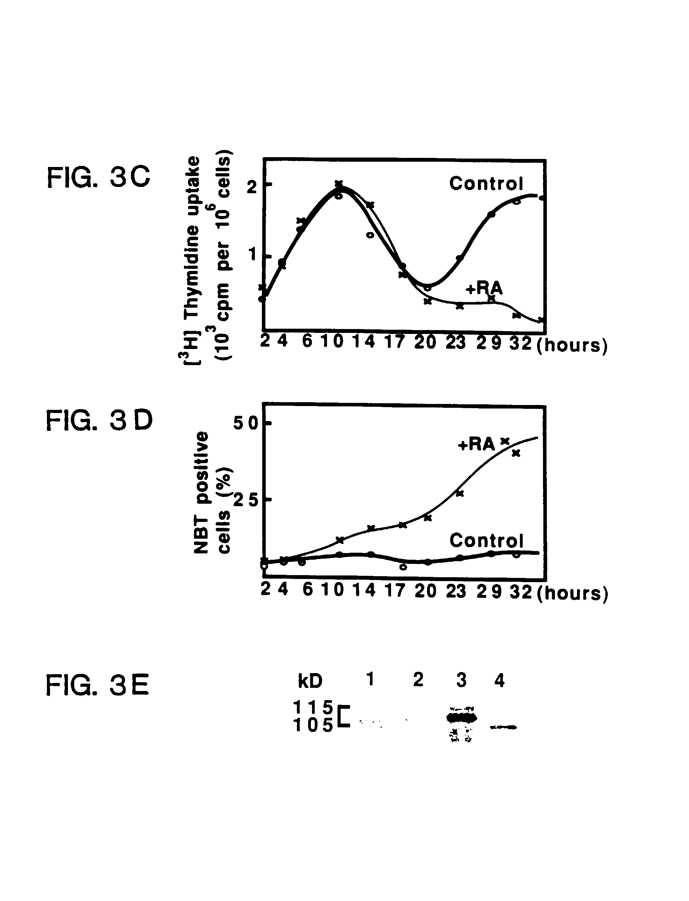

Fig.3 demonstrates the Rb phosphorylation in HL-60A cells induced to terminally differentiate by various chemicals. (A) A random population of exponentially proliferating HL-60A cells in RPMI 1640, 10% FCS were treated with either 5 X 10⁻⁶ RA (O), or 1.25% DMSO (Yen et. al., Exp. Cell Res. 168, 247 (1987) for different lengths of time. Cell numbers were counted at zero time and at 24 hour intervals. The results are shown in Figure 3A. The untreated control sample is represented by X, RA treated samples by o, and DSMO samples by +.

### Example 5

A duplicate culture of HL-60A cells from Example 3 was labeled with 300 Uci of [³⁵S]-methionine (1000 Ci/mmol) and analyzed as described in Example 1A. The cells were treated with 5 X 10⁻⁶ M RA (lanes 1 to 6), 1.25% DMSO (lane 7 to 9) or 4 X 10⁻³ M BA (lane 10). Metabolic labeling of cells was at time 0 (lane 1 ), 12 hours (lane 2), 24 hours (lane 3), 48 hours (lane 4), 72 hours (lane 5), 96 hours (lane 6), 12 hours (lane 7), 24 hours (lane 8), 96 hours (lane 9), and 96 hours (lane 10). The arrow points to a species of Rb of about 98 kD that appeared reproducibly in cells treated with the above chemicals for 96 hours.

(C) HL-60 cells at G₀ and G₁ phases were isolated by centrifugal elutriation from an actively proliferating cell population. Centrifugal elutriation was performed in a Beckman J21-C centrifuge with a JE-6 rotor containing a 40 ml chamber. 10⁹ cells were loaded into the chamber. The rotor speed was kept at 2000 rpm and the flow rate was increased from an initial rate of 11.5 ml/minute to a final of 29 ml/minute in 12 increments. Go and G₁ cells were collected at 14.3 ml/minute and their size monitored under the microscope. Duplicate cultures of the collected samples were grown in RPM1 1640 plus 10 % FCS in the presence (X) or absence (0) of 5 X 10⁻⁶ M RA for 48 hours. DNA synthesis was measured by [³H]-thymidine uptake as described by Ludlow et.al., Cell 56: 57 (1989).

### Example 6

A small sample was taken at each time point in Example 5 to analyze for cell differentiation by uptake of NBT as described by Collins et. al., Int J. Cancer 25: 213 (1980). The results are shown on Figure 3D. NBT-positive cells were expressed as a percentage of the total number of cells counted, (X), RA treated cells; (0), control. Duplicate samples of Gₒ and G₁ HL-60A cells from experiments described above were used for analysis of Rb expression. Harvested cells were treated with RA at time O and Rb analysis was performed at approximately 0, 4, 8 and 18 hours. The results are shown on Figure 3E. Cells were harvested at 2 1/2 hours (lane 1), 6 1/2 hours (lane 2), 10 1/2 hours (lane 3) and 20 1/2 hours (lane 4) after collection. Since it is not possible to separate the early and the late G₁ cells, some cells have traversed the G₁/S boundary during the period of labeling.

Multiple forms of Rb, with apparent molecular masses from 105 to 115 Kd, were detected by immunoprecipitation from the human bladder tumor cell line VM-CUB-3 labeled with [³⁵S]-methionine, followed by SDS-polyacrylamide gel electrophoresis (SDS PAGE) (Fig. 1A, lane 1). No such protein could be precipitated when the antibodies were pre-absorbed with the corresponding Rb peptides (Fig. 1A lane 2). Metabolic labeling with [³²P]-orthophosphate demonstrated that the Rb polypeptides with higher apparent molecular mass were more heavily phosphorylated (Fig.1 A, lane 3). Treatment of the phosphoproteins with potato acid phosphatase prior to immunoprecipitation resulted in the disappearance of these Rb species.
Immunohistochemical staining of cells using these antibodies revealed the nuclear localization of Rb as has been described (Lee, et. al., Nature 329,642(1987); Varley et. al., Oncogene 4, 721 (1989)) and is predicted from the presence of a stretch of amino acids VRSPKKK (amino void residues 609 to 615) reminiscent of the nuclear translocation signal found in SV40 large T antigen. (Abbreviations for the amino acid residues are: A, Ala; C, Cys; D, Asp; E, Glu; F, Phe; G, Gly; H, His; I, Ile; K, Lys: L, Leu; M, Met; N, Asn; P, Pro; Q, Gln; R, Arg; S, Ser; T, Thr; V, Val; W, Trp; and Y, Tyr.)

### Example 7

The synthesis and degradation of Rb is regulated in a cell cycle-dependent manner. The steady-state amount of Rb as a function of the cell cycle was measured. Because the rabbit polyclonal antibodies Rb 1-AB 20, Rb 1 AB 16, and Rb 1-AB-A1 detect the various phosphorylated forms of Rb, the total amount of Rb in cells was measured. Exponentially proliferating wild-type HL-60 cells were fixed and stained with Rb 1-AB 20 and a secondary fluorescein-conjugated rabbit antibody to immunoglobulin G (IgG). The cells were then treated with ribonuclease A (Rnase A) and stained with propidium iodide for quantitation of DNA. Because fluorescein emission is green and propidium iodide emission is red when the cells were irradiated at 488 nm, the simultaneous measurement of the emission intensities, and therefore the Rb and DNA content, was determined by flow cytometry (Fig. 1B). The content of Rb per cell increased as the cells progressively matured through the G₁, S, and G₂+M phases of the cell cycle. Cells in the G₂+M phases contain approximately twofold more Rb than cells entering G₁. Therefore, the Rb content of a proliferating cell is always maintained at or above the threshold level in G₀ and G₁. This would be expected if the rate of Rb synthesis exceeds that of its degradation. These results were reproduced when the experiments were performed with Rb 1-AB A1.

To ascertain the dynamics of Rb turnover, the synthesis and degradation of Rb were studied by immunoprecipitation. The human breast tumor cell SW613 was pulse-labeled for 90 min. with [³⁵S]-methionine and the degradation rate of labeled Rb was monitored (Fig. 1C). Densitometric scanning of the intensities of the various forms of Rb showed the half-life of Rb to be at least 10 hours. A similar Rb half life profile has been observed by others (Whyte, et al., Nature 334, 124 (1988); Xu, et al., Oncogene 4, 807 (1989)).

The synthesis of Rb as a function of the cell cycle in SW613 and VM-CUB-3 is demonstrated in Fig. 2. Two to three days after the withdrawal of serum, the exponentially growing cells ceased to proliferate. Cell numbers remained constant and no DNA synthesis occurred during the next five days. When these cells were released into the proliferative phase by the addition of serum, or with EGF plus insulin and transferrin alone, they synchronously traversed the cell cycle. These cells were pulse-labeled with [³⁵S]-methionine at regular intervals and Rb immunoprecipitated for analysis. Under these conditions Rb was found to be synthesized in both the quiescent state (Fig. 2A, lanes 1 to 5, Fig. 2B, lane 1), as well as in the proliferative phases (Fig.2B, lanes 3 to 13). The fact that Rb is stable and is constantly being synthesized is consistent with the observed steady-state accumulation of the protein as the cell volume increases.

The inability of a cell to down modulate its Rb content suggested that it must have other ways of regulating the activity of this protein. In the quiescent state, the cells had only a single species of underphosphorylated Rb with an apparent molecular mass of 105 KD (Fig. 2A, lanes 3 to 5, Fig. 2B, lane 1). Upon reintroduction of serum, the synchronized cells traversing the cell cycle still exhibited only the underphosphorylated form of Rb at the early G₁ phase (Fig. 2B, lanes 3 and 4). However cells at the G₁/S boundary and in the S phase (Fig. 2B, lanes 5 to 10), regained the ability to produce multiply phosphorylated newly synthesized Rb. Ongoing DNA synthesis is not required for the cells to produce multiply phosphorylated Rb. Cells arrested at the G₁/S boundary with aphidicolin for as long as 24 hours were fully competent in producing and maintaining the newly synthesized Rb in its multiply phosphorylated state (Fig. 2B, lane 13). At the G₂+M phase, the underphosphorylated forms of Rb again became more prominent (Fig.2B, lanes 11 and 12). However, cells arrested at the M phase with 3-(1-Anilinoethylidene)-5-benzylpyrrolidine-2,4-dione (WAKO Chemicals U.S.A., inc.) still exhibited more than one form of Rb. The fact that Rb became heavily phosphorylated just before the onset of DNA synthesis suggests a possible correlation between the state of Rb phosphorylation and the control of cell proliferation.

The effect of the abnormal growth conditions imposed by serum starvation on the phosphorylation profile was compared to the phosphorylation profile of Rb in cells induced to undergo terminal differentiation. HL-60 cells can be induced to terminally differentiate along several different lineages by treatment with various chemicals. Treatment with retinoic acid (RA) or dimethyl sulfoxide (DMSO) induces myelocytic differentiation, whereas 1,25-dihydroxyvitamin D3 and 12-O-tetradecanoyl phorbol-13-acetate (TPA) induce monocytic differentiation. The induction process typically occurs over a period of time corresponding to two cell cycles (Yen, et. al., Leukemia Res. 10, 619 (1986)). In the first cell cycle, cells exposed to inducers develop a precommitment state. The cells at this stage are not committed to either growth arrest or differentiation and they can continue to proliferate indefinitely.

We have isolated a variant HL-60 cell line (HL-60A) that has developed this precommitment state. Exposure of HL-60A to an inducer for a single cell cycle is enough to trigger cell arrest and onset of differentiation. The relationship between the control of cell proliferation and the state of phosphorylation of Rb was determined by treating a random population of actively proliferating HL-60A cells with RA. The cell number doubled in the first 24 hours of treatment with RA (Fig 3A). Inhibition of proliferation thereafter occurred since no further increase in cell number was observed. As early as 12 hours after treatment, down modulation of Rb phosphorylation was observed (Fig.3B, lanes 2 and 7). After 24 hours of treatment with RA or DMSO, virtually all of the newly synthesized Rb was underphosphorylated (lanes 3 and 8).

However, these data alone do not readily distinguish whether the change in Rb phosphorylation profile is a cause or a consequence of growth arrest. It is possible that RA treatment can directly down modulate the degree of Rb phosphorylation in all cells, and thereby lead to growth arrest. Alternatively, down modulation of the phosphorylation of Rb may occur only at a restricted point of the cell cycle, for example, at the G₀ and G₁ phases. Down modulation of Rb phosphorylation in the latter case would therefore be a reflection of the fact that the cells are growth arrested at G₀ and G₁ and the observed accumulation of underphosphorylated Rb after RA treatment may represent the gradual arrival of the cells at the restriction point. To address this question, we performed the following experiments. We first isolated a synchronous G₀/G₁ population of HL-60A cells by centrifugal elutriation. After isolation, cells were divided into two portions, one of which was treated with RA. At regular intervals, cell samples were taken for analysis of the Rb phosphorylation profile by immunoprecipitation and for measurement of proliferation by [³H]-thymidine uptake. The synchronized HL-60A cells were able to grow in the presence of RA for one cell division before the onset of growth arrest (Fig.3C). During this cycle, the newly synthesized Rb was phosphorylated as the cells moved into the S phase, in a manner indistinguishable from that of untreated cells (Fig. 3E, lanes 1 to 4). At 30 hours after treatment, the RA-treated cells became growth arrested, and also lost the ability to produce multiply phosphorylated Rb (Fig.3E, lane 5). A significant percent of the cells become differentiated as measured by nitrobluetetrazolium (NBT) staining Fig. 3D). We conclude that RA treatment does not directly modulate the phosphorylation of the protein. Rather, down modulation of the phosphorylation of newly synthesized Rb is a consequence of RA-induced growth arrest. This conclusion is supported by the observation that treatment of HL-60A cells with compounds such as TPA, DMSO, or sodium butyrate (BA) all resulted in a similar time course of down modulation of Rb phosphorylation (Fig. 3B, lanes 7 to 10), despite the fact that these compounds each affect a different cellular target. After 96 hours of treatment with either RA, BA or DMSO, the cells exhibited an underphosphorylated Rb protein of 98 KD. Since Northern blot analysis revealed a normal size MRNA, the origin of this truncated Rb could be due to the initiation of translation at an internal ATG. As this species of Rb is observed in cells treated with the various chemical inducers, its appearance may be related to the state of growth arrest rather than differentiation of the cells.

The phosphorylation of the retinoblastoma protein is central to the control of its activity. A partially purified preparation of the Rb kinase that can phosphorylate the Rb protein contains the CDC 2 kinase complexed with cyclin B. The CDC 2/cyclin B complex phosphorylates the Rb protein in vitro. Most of the phosphorylation sites that can be utilized by this enzyme in vitro are also used in vivo.

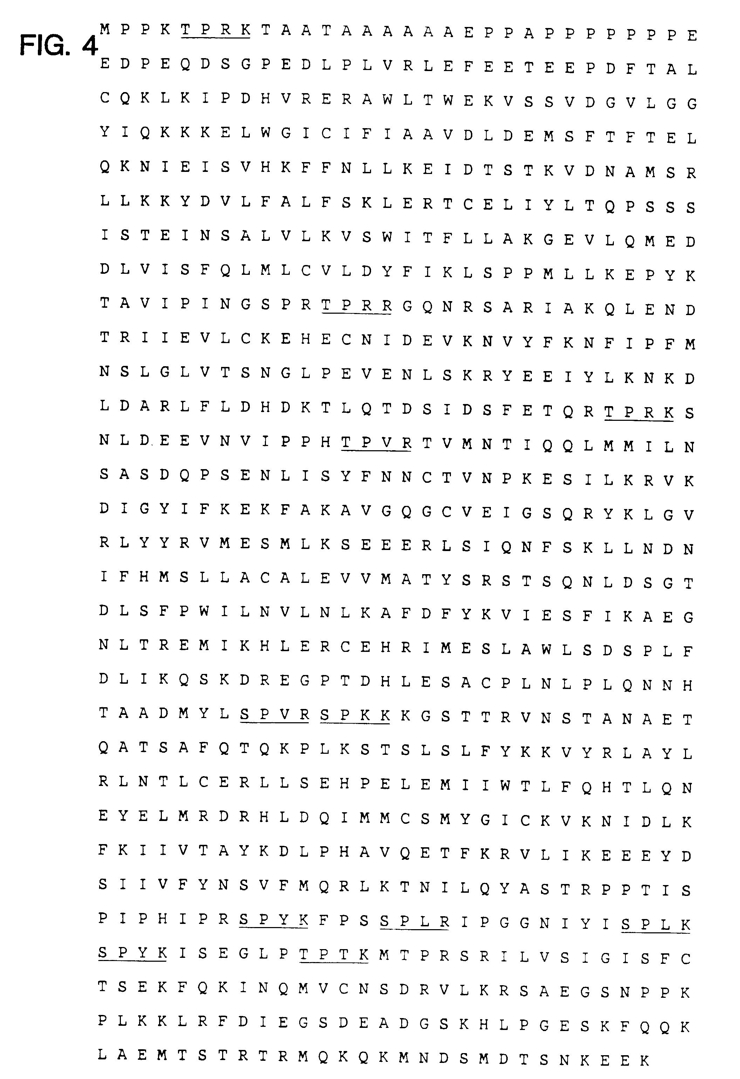

The phosphorylation sites in the Rb protein that contain the motif S/TPXY where Y can be either K, R or H are recognized by the CDC 2 kinase. There are 11 such potential phosphorylation sites in the Rb protein (Fig. 4, underlined). Some of them must be important for the regulation of the activity i.e. if these important sites are phosphorylated, the Rb protein would be rendered inactive.

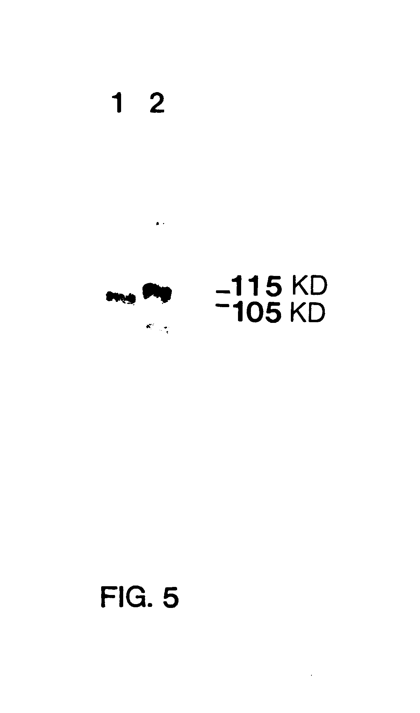

There are at least two ways to distinguish between the phosphorylated form of the Rb protein (inactive form) and the underphosphorylated form (active) of the Rb protein. One method is by immunoprecipitation whereby cells in tissue culture are labeled with either ³⁵S-methionine or ³²P. The Rb protein is then extracted and precipitated by incubation with an antibody against the Rb protein. The underphosphorylated form of the Rb protein which has a lower molecular weight (approx. 105 kilo dalton) than the heavily phosphorylated form and can be distinguished by gel electrophoresis. Another way whereby the underphosphorylated form of the Rb protein can be identified is by using immunohistochemical staining of tissue sections or culture cells. Antibodies against an unphosphorylated peptide which contains the phosphorylation site may only recognize the Rb protein containing an unphosphorylated site. One such antibodies, OS3, is specific for the peptide GSPRTPRRGQNRSAR, the region of the Rb peptide which contains the potential phosphorylation site for the CDC 2 kinase from amino acid 248 to 262. (Figure 4). Antibody against this peptide detects only the underphosphorylated form of the Rb protein, as shown in Figure 5 lane 1, where the molecular weight of the Rb protein immunoprecipitated with OS3 is about 105 kd. In contrast, an antibody OS1, directed against another part of the Rb protein, that does not have that phosphorylation site detects the underphosphorylated as well as the heavily phosphorylated form of the Rb protein as is shown in Figure 5 lane 2. Some phosphorylation sites, for example the one we just cited, are not randomly phosphorylated, meaning the absence of phosphorylation of this site is always associated with the underphosphorylated form of the Rb protein (active form of Rb protein). An antibody against this and/or other similar sites will distinguish between the phosphorylated and underphosphorylated form of the Rb protein, either by immunoprecipitation or by immunohistochemical staining of uncultured tissue sections. Antibody raised against these phosphorylation sites, or in the vicinity will be useful for such studies, especially for immunohistochemical staining. These sites are underlined in Fig. 4. Oligopeptides of 15 amino acid residue or more containing these sites were used to raise the site specific antibody.

Many therapeutic reagents for growth arrest and differentiation for example, interferon, (alpha, beta, gamma), tumor necrosis factor and tumor growth factor β can induce the differentiation and growth arrest of many different cell types, such as leukemia cells and solid tumors. One common action of these drugs is the down regulation of the Rb protein phosphorylation.

When the Rb gene was introduced into various types of tumor cells, the growth of the cells was inhibited within one or a few cell cycles often at the single cell level. This data suggests that the anti-proliferative effect is an intrinsic effect of the Rb protein. The transfection essentially led to the production of an excess amount of the active form of the Rb protein, which cannot be dephosphorylated by the cell, and the presence of such active protein was sufficient to stop the cells from growing. Many of the therapeutic drugs led to the down regulation of Rb protein phosphorylation and thereby activate the Rb protein.

The effectiveness of a given regimen of drug treatment can be assessed by monitoring the phosphorylation status of the Rb protein. This assessment is particularly useful in providing a rapid method of determining whether a particular drug is effectively inhibiting abnormal cell proliferation in cells containing the Rb gene. Tumor cell specimens are removed during and after drug treatment and the degree of Rb protein phosphorylation is determined. Whether a given drug inhibited Rb protein phosphorylation when given alone or in combination with other drugs can be determined by a number of methods. Tumor cells can be biopsied or removed from the patient and subjected to the treatment of a given drug or combination of drugs in vitro. The phosphorylation of the Rb protein is measured by immunoprecipitation and/or immunohistochemical staining at various times after treatment to assess whether Rb phosphorylation has been inhibited. If the phosphorylation was not inhibited in vitro, the treatment is unlikely to lead to cell growth arrest. On the other hand, if the drug effectively inhibits the phosphorylation of the Rb protein in vitro, there is a high probability that the treatment will be successful in the patient.

Alternatively, during the treatment of the patient, tumor samples can be biopsied and subjected to immunohistochemical staining without culturing, using antibody against specific phosphorylation sites, to assess the effectiveness of the drug treatment and whether the down regulation of Rb protein phosphorylation has been induced.

The endpoint of any assay of the effectiveness of drug treatment would be a determination of the ability of the subject drug to inhibit Rb protein phosphorylation at the important sites.

## Claims

1. A method of identifying drugs which inhibit abnormal cell proliferation comprising incubating said drug with abnormally proliferating cells containing Rb protein for a period of time sufficient to allow phosphorylation of said Rb protein, and measuring the extent of underphosphorylation of said Rb protein in said cells.

2. The method of claim 1 wherein said incubation period is 12 hours.

3. A method of determining whether an abnormally proliferating cell will be growth inhibited in response to a drug comprising incubating said drug with abnormally proliferating cells containing Rb protein for a period of time sufficient to allow phosphorylation of said Rb protein, and measuring the extent of underphosphorylation of said Rb protein in said cells.

4. A method for determining the effectiveness of a drug for the treatment of a cell proliferative disease comprising:
a) treating a portion of a cell sample obtained from an individual prior to treatment with the drug, with said drug in vitro;
b) measuring the level of Rb protein phosphorylation in the portion of the cell sample treated in step (a);
c) measuring the level of Rb protein phosphorylation in an untreated portion of the cell sample;
d) comparing the level of Rb protein phosphorylation from said cell sample portion of step (b) with the level of Rb protein phosphorylation from said cell sample portion of step (c),
wherein a drug which inhibits Rb protein phosphorylation is thereby assessed to be effective in treating cell proliferative disease.

5. The method of claim 4, wherein the measurement of Rb protein phosphorylation is by immunoprecipitation.

6. The method of claim 4, wherein the measurement of Rb protein phosphorylation is by immunohistochemical staining.

7. The method of claim 4, wherein the measurement of Rb protein phosphorylation is by precipitating Rb protein with antibodies specific for the phosphorylation sites of said Rb protein.

8. The method of claim 4, wherein the degree of Rb protein phosphorylation is determined by precipitating Rb protein with an antibody which binds to Rb protein, subjecting the Rb protein to gel electrophoresis and determining the degree of Rb phosphorylation by comparison with molecular weight standards.

## Patentansprüche

1. Verfahren zur Identifizierung von Arzneimitteln, die eine anormale Zellvermehrung hemmen, umfassend, daß man das Arzneimittel mit anormal sich vermehrenden Zellen, die Rb-Protein enthalten, über einen Zeitraum inkubiert, der ausreicht, um die Phosphorylierung des Rb-Proteins zuzulassen und das Ausmaß der Unterphosphorylierung des Rb-Proteins in diesen Zellen mißt.

2. Verfahren nach Anspruch 1, worin der Inkubationszeitraum 12 Stunden ist.

3. Verfahren zur Bestimmung, ob das Wachstum einer anormal sich vermehrenden Zelle gehemmt wird als Reaktion auf ein Arzneimittel umfassend, daß man das Arzneimittel mit anormal sich vermehrenden Zellen, die Rb-Protein enthalten, über einen Zeitraum inkubiert, der ausreicht, um eine Phosphorylierung des Rb-Proteins zuzulassen und das Ausmaß der Unterphosphorylierung des Rb-Proteins in diesen Zellen mißt.

4. Verfahren zur Bestimmung der Wirksamkeit eines Arzneimittels zur Behandlung einer zellproliferativen Krankheit umfassend, daß man:
a) einen Teil einer Zellprobe, die von einem Patienten vor der Behandlung mit dem Arzneimittel erhalten wurde, mit dem Arzneimittel in vitro behandelt;
b) den Grad der Rb-Protein-Phosphorylierung in dem Teil der in Stufe (a) behandelten Zellprobe mißt;
c) den Grad der Rb-Protein-Phosphorylierung in einem nicht behandelten Teil der Zellprobe mißt;
d) den Grad der Rb-Protein-Phosphorylierung von dem Zellprobenteil von Stufe (b) mit dem Grad der Rb-Protein-Phosphorylierung von dem Zellprobenteil von Stufe (c) vergleicht,
wodurch für ein Arzneimittel, das die Rb-Protein-Phosphorylierung hemmt, festgestellt wird, daß es wirksam ist zur Behandlung einer zellproliferativen Krankheit.

5. Verfahren nach Anspruch 4, worin die Messung der Rb-Protein-Phosphorylierung durch Immunfällung erfolgt.

6. Verfahren nach Anspruch 4, worin die Messung der Rb-Protein-Phosphorylierung durch immunhistochemische Färbung erfolgt.

7. Verfahren nach Anspruch 4, worin die Messung der Rb-Protein-Phosphorylierung durch Ausfällung von Rb-Protein mit Antikörpern, die spezifisch für die Phosphorylierungsstellen des Rb-Proteins sind, erfolgt.

8. Verfahren nach Anspruch 4, worin der Grad der Rb-Protein-Phosphorylierung bestimmt wird, indem Rb-Protein mit einem Antikörper ausgefällt wird, der an Rb-Protein bindet, das Rb-Protein einer Gelelektrophorese unterzogen wird und der Grad der Rb-Phosphorylierung durch Vergleich mit Molekulargewichtsstandards bestimmt wird.

## Revendications

1. Procédé d'identification de médicaments qui inhibent une prolifération cellulaire anormale, comprenant l'incubation dudit médicament avec des cellules proliférant anormalement contenant une protéine Rb, pendant une durée suffisante pour permettre la phosphorylation de ladite protéine Rb et la mesure de l'importance de la sous-phosphorylation de ladite protéine Rb dans lesdites cellules.

2. Procédé selon la revendication 1, dans lequel ladite période d'incubation est de 12 heures.

3. Procédé de détermination du fait qu'une cellule proliférant anormalement aura sa croissance inhibée en réponse à un médicament, comprenant l'incubation dudit médicament avec des cellules proliférant anormalement contenant une protéine Rb, pendant une durée suffisante pour permettre la phosphorylation de ladite protéine Rb et la mesure de l'importance de la sous-phosphorylation de ladite protéine Rb dans lesdites cellules.

4. Procédé de détermination de l'efficacité d'un médicament destiné au traitement d'une maladie de prolifération cellulaire, comprenant :
a) le traitement d'une portion d'un échantillon de cellules, obtenu à partir d'un individu avant traitement par le médicament, avec ledit médicament in vitro ;
b) la mesure du niveau de phosphorylation de la protéine Rb dans la portion de l'échantillon de cellules traitée à l'étape (a) ;
c) la mesure du niveau de phosphorylation de la protéine Rb dans une portion non-traitée de l'échantillon de cellules ;
d) la comparaison du niveau de phosphorylation de la protéine Rb de ladite portion de l'échantillon de cellules de l'étape (b) avec le niveau de phosphorylation de la protéine Rb de ladite portion de l'échantillon de cellules de l'étape (c),
dans lequel un médicament qui inhibe la phosphorylation de la protéine Rb est ainsi estimé être efficace dans le traitement d'une maladie de prolifération cellulaire.

5. Procédé selon la revendication 4, dans lequel la mesure de phosphorylation de la protéine Rb s'effectue par immunoprécipitation.

6. Procédé selon la revendication 4, dans lequel la mesure de phosphorylation de la protéine Rb s'effectue par coloration immunohistochimique.

7. Procédé selon la revendication 4, dans lequel la mesure de phosphorylation de la protéine Rb s'effectue par précipitation de la protéine Rb avec des anticorps spécifiques pour les sites de phosphorylation de ladite protéine Rb.

8. Procédé selon la revendication 4, dans lequel le degré de phosphorylation de protéine Rb est déterminé par précipitation de protéine Rb avec un anticorps se liant à la protéine Rb, soumission de la protéine Rb à une électrophorèse de gel et détermination du degré de phosphorylation de Rb par comparaison avec des normes de poids moléculaires.
